# EUROPEAN PATENT SPECIFICATION

(11) **EP 2 763 593 B1**
(45) Date of publication and mention of the grant of the patent: **09.05.2018**
(21) Application number: 12829774.4
(22) Date of filing: 10.09.2012
(51) Int. Cl.: A61B 17/34, A61B 90/00, A61B 5/00, A61B 17/02, A61B 17/00

(54) **RIB-PROTECTING DEVICES FOR THORACOSCOPIC SURGERY**
RIPPENSCHUTZVORRICHTUNG FÜR CHIRURGISCHE THORAX-EINGRIFFE
DISPOSITIFS POUR LA PROTECTION DES CÔTES POUR LA CHIRURGIE THORACOSCOPIQUE

(30) Priority: 08.09.2011 US 201161573583 P; 23.05.2012 US 201261687585 P
(43) Date of publication of application: 13.08.2014
(73) Proprietor: Physcient, Inc., Durham, NC 27705 (US)
(72) Inventor: PELL, Charles Anthony, Durham, NC 27701 (US); CRENSHAW, Hugh Charles, Durham, NC 27705 (US); ESPENHAHN, Eric Torr, Cary, NC 27519 (US)
(74) Representative: Dolleymores
(86) International application number: PCT/US2012/054442
(87) International publication number: WO 2013/036921

(56) References cited:
- WO-A2-2009/126953
- US-A1- 2003 216 619
- US-A1- 2004 073 301
- US-A1- 2005 043 592
- US-A1- 2009 259 109

## Description

### Field of the Disclosure

The invention relates to a rib-protecting clip assembly for thoracoscopic surgery.

### Background

Deformation of tissues is commonly performed during surgery or other medical procedures either to achieve surgical access or to specifically alter the dimensions of one part of the anatomy. Examples of deformations of tissue for surgical access include spreading ribs during a thoracotomy, spreading a bisected sternum during a sternotomy, and separating the vertebrae of the spine for surgery on the intervertebral disk. Examples of deformation of tissues to alter the dimensions of the tissue include angioplasty to open blocked arteries, valvuloplasty to enlarge heart valves, and distraction to adjust the position of vertebrae. Such deformations are collectively referred to herein as "retraction".

Spreaders, retractors, distractors, and even angioplasty balloons (collectively called "retractors" here) can impose significant forces on the surrounding tissues during retraction. The resulting strain on these tissues, and on associated tissues such as the ligaments attaching ribs to vertebrae, can be large, leading to damage of these tissues, including the fracture of ribs and the rupture or irreversible deformation of ligaments and other fibrous tissues.

Retraction occurs in two different phases -deforming the tissue (referred to herein as the first phase or retraction) and holding the tissue at that deformation (referred to herein as the second phase of retraction). Usually both are done with the same instrument. For example, a rib spreader is used both to force the ribs apart during a thoracotomy and to hold the ribs apart during the surgical procedure. Sometimes two different instruments are used, especially if the deformation is to be permanent. For example, an angioplasty balloon is used to force open an atherosclerotic plaque, and then a stent is used to hold the artery open; or a distractor is used to separate vertebrae, and a metal plate is used to secure the vertebrae in that position. An example of two different instruments being used when the deformation is not permanent is disclosed in US Pat. App. No. 5,201,325 by McEwen (McEwen, Auchinleck et al. 1993), therein a surgeon manually retract an incision with a disclosed retractor blade, and an automated mechanism is then used to hold the incision open. In the medical literature, both phases are frequently referred to as retraction.

Both phases of retraction traumatize tissue. Trauma from the first phase of retraction can include the rending and tearing of tissues - bones bend and break; muscles stretch beyond normal limits; ligaments and other connective tissues stretch and tear; or nerves are stretched. Trauma from the second phase of retraction can include ischemia of the tissue due to elevated tissue pressure, for example, under a retractor blade; blockage of nerves; and blockage of blood vessels causing ischemia in tissues distant from retraction.

Tissue trauma and ensuing complications resulting from retraction can be greater than the trauma resulting from the medical procedure that required the retraction. For example, thoracotomies are extremely traumatic, and can result in post-surgical pain and respiratory complications that exceed that of the thoracic procedure, such as a lung segmentectomy.

WO 2009/126953A discloses a retractor having clips for engaging and retracting ribs.

There is a need for improved devices for use in thoracoscopic surgery

### Summary of the Detailed Description

According to the present invention there is provided a rib-protecting clip assembly having the features recited in the attached claim 1. The invention provides a device adapted to protect the tissues surrounding the tissue, especially the ribs, from trauma arising from impingement by surgical instruments as the surgeon operates.

In one non-limiting embodiment, a device comprises a rib-protecting clip for thoracic surgical access through an intercostal incision. The clip comprises at least one rib-engaging channel. The at least one rib engaging channel comprises a first end of the at least one rib-engaging channel, a second end of the at least one rib-engaging channel, and at least one raised member. The at least one raised member is disposed at or between the first end of the at least one rib-engaging channel and the second end of the at least one rib-engaging channel. The at least one raised member is further elevated from the at least one rib-engaging channel toward the rib. The at least one raised member is further disposed along a portion of the at least one rib-engaging channel such that at least one rib-contacting surface on the at least one raised member contacts the rib, and the rest of the at least one rib-engaging channel does not contact the rib. In this manner, the rib-engaging channel thus prevents impingement by surgical instruments onto the rib.

### Brief Description of the Drawings

FIG. 1 shows the anatomy of a thoracic incision.
FIG. 2A shows a device for retracting tissue around a thoracic incision.
FIG. 2B shows an oblique view of the above device.
FIG. 2C shows a side view of the above device.
FIG. 2D shows an oblique view of a modified version of the above device.
FIG. 2E shows a side view of the above device.
FIG. 3A shows an oblique view of a clip for retracting thoracic tissue.
FIG. 3B shows a sequential view of the above clip in side view.
FIG. 3C shows a sequential view of the above clip being attached to a rib.
FIG. 3D shows an oblique view of another clip for retracting thoracic tissue.

### Detailed Description

The invention is directed to devices for gaining surgical access for thoracoscopic surgery or minimally invasive surgery, permitting instruments to be introduced between ribs into the chest without damaging the surrounding tissues, including the intercostal nerve and vasculature.

Devices in accordance with the invention are smaller and simpler than conventional retractors used for thoracoscopic surgery with only a few moving parts that fit almost entirely inside the intercostal incision, in most cases leaving no parts projecting out of the skin incision. Despite being smaller and simpler, these devices still manage to protect the surface of the rib and the intercostal neurovascular bundle from damage by preventing impingement of surgical instruments inserted between the cranial and caudal ribs, impingement that can damage fragile tissues and lead to post-surgical pain and complications.

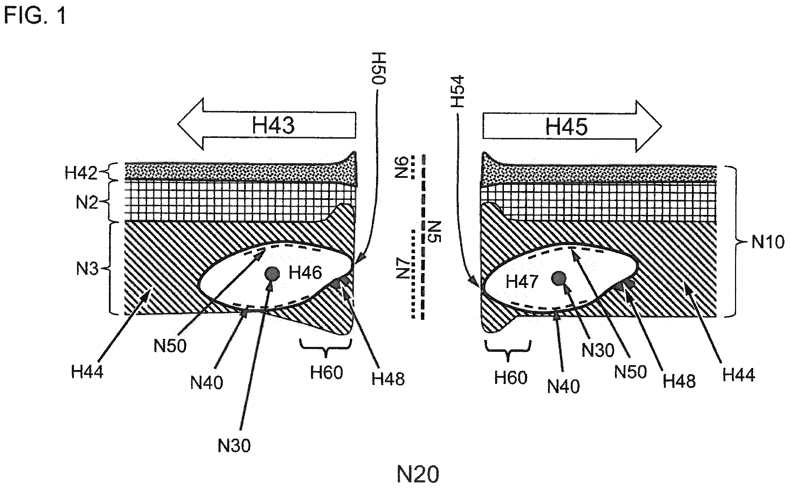

FIG. 1 establishes the relevant anatomy, illustrating an intercostal incision, in cross-section, passing through the tissues of the thorax, between adjacent ribs, and into the pleural cavity. FIG. 1 is presented solely to identify the appropriate anatomy and incision and does not present any inventions. The tissues of the thoracic wall N10 include the skin H42, subcutaneous tissues N2, and a layer N3 containing ribs H46 and H47 and intercostal muscle H44. The space interior to the thoracic wall N10 is the pleural cavity N20. The patient's head is in the cranial direction H43 and the feet are in the caudal direction H45. A thoracic wall incision N5 is made through the thoracic wall N10 passing between ribs H46 and H47, such that one rib is designated the cranial rib H46 having a caudal margin H50 adjacent the thoracic wall incision N5, and the opposing rib is the caudal rib H47 having a cranial margin H54 adjacent the thoracic wall incision N5. Each rib H46 and H47 has a longitudinal axis N30 (shown here coming out of the page), a pleural surface (dashed line) N40 facing pleural space N20, and a skin surface N50 facing skin H42. A neurovascular bundle H48 lies just inside and below (pleurally) the caudal-most edge of each rib H46 and H47. The neurovascular bundle H48 contains the intercostal nerve and intercostal vasculature. For purposes of this discussion, the thoracic incision N5 through all the tissues of the thoracic wall N10 can be sub-divided into the skin incision N6 through the skin H42 and the intercostal incision N7 through the intercostal muscle H44.

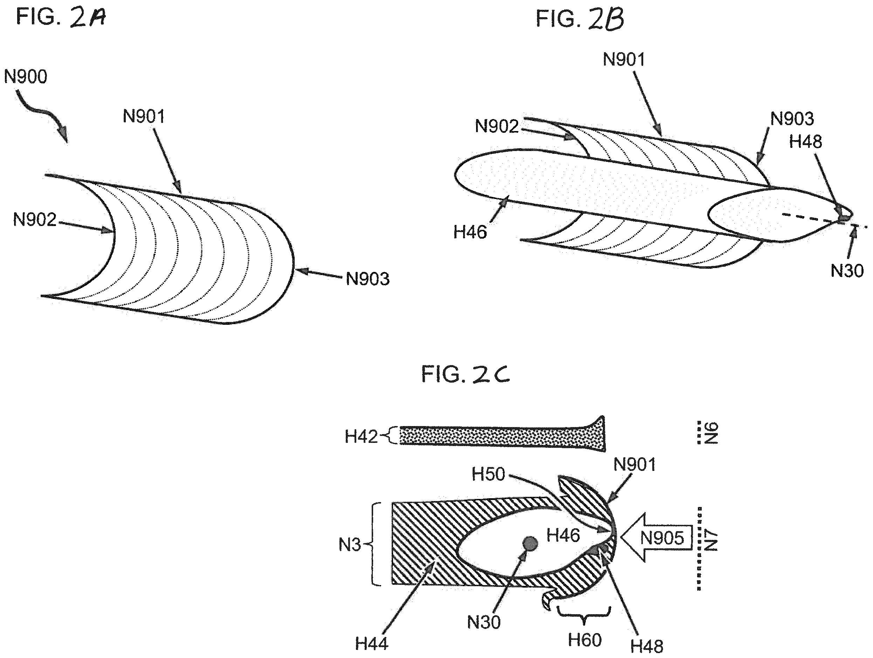

FIGS. 2A through 2E shows a rib-protecting clip N900 that resides solely in the intercostal incision N7 with no part projecting through the skin incision N4 and thus no part above the skin H42. FIGS. 2A through 2E show rib-protecting clip N900 apposed to the caudal margin H50 of a cranial rib H46, but rib-protecting clip N900 can attach to the cranial margin H54 of the caudal rib H47 in similar fashion.

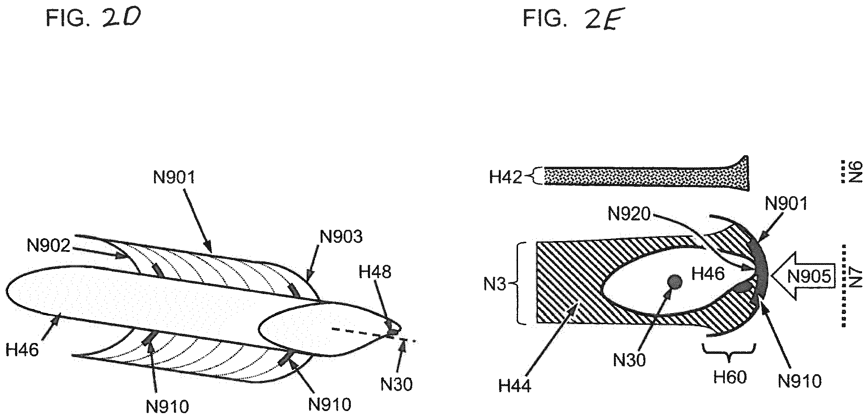

FIG. 2A shows an oblique view of the rib-protecting clip N900. FIG. 2B shows the rib-protecting clip N900 with a rib, here cranial rib H46. FIG. 2C shows a cross-sectional view, including select anatomy around the incision. Rib-protecting clip N900 comprises a rib-engaging channel N901 having a first end N902 and a second end N903 and engages cranial rib H46, generally along longitudinal axis N30 of the cranial rib H46, wrapping around the caudal margin H50 of cranial rib H46. Rib-engaging channel N901 thus serves as a barrier to prevent impingement of any object that is inserted into intercostal incision N7 onto cranial rib H46. When an object, such as a surgical instrument, is inserted into intercostal incision N7, rib-protecting clip N900 prevents direct impingement by that object onto cranial rib H46, thereby protecting cranial rib H46. Nevertheless, that object can exert a force N905 that pushes rib-engaging channel N901 into cranial rib H46, thereby crushing intercostal muscle H44 and other associated tissues into rib H46 and creating a high pressure region H60, all of which can potentially damage these tissues and the neurovascular bundle H48. FIGS. 2D and 2E show modifications in accordance with the present invention that reduce this broad crushing of intercostal muscle H44 and the neurovascular bundle H48. FIGS. 2D and 2E show identical views as FIGS. 2B and 2C, respectively, but now rib-engaging channel N901 sports raised members N910 that are disposed along a portion of rib-engaging channel N901 between first end N902 and second end N903 and that are elevated from rib-engaging channel N901 toward cranial rib H46. Raised members N910 here are depicted as raised ridges on rib-engaging channel N901 that place loading from force N902 onto discrete rib-contacting surfaces, seen in FIG. 2E as rib-contacting surface N920. Other than rib-contacting surfaces N920, no other portion of rib-protecting clip N900 contacts cranial rib H46. Raised members N910 thus relieve pressure on (and damage to) intercostal muscle H44 and neurovascular bundle H48 caused by the rest of rib-engaging clip N900.

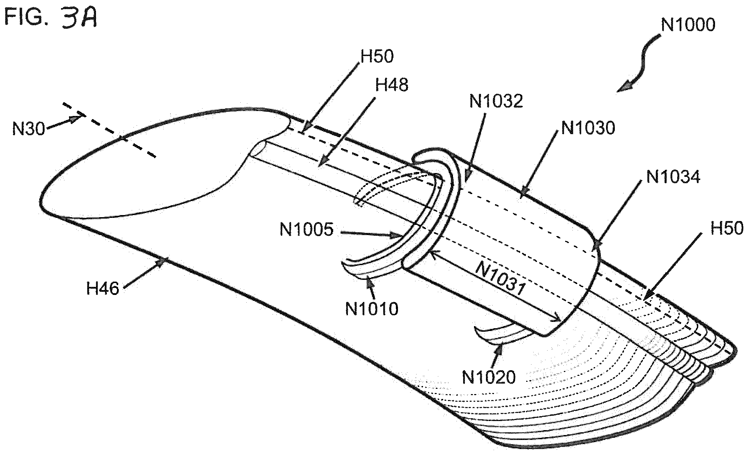

FIGS. 3A through 3C shows another rib-protecting clip N1000 with components to attach reversibly to a rib. FIGS. 3A through 3C show rib-protecting clip N1000 apposed to the caudal margin H50 of a cranial rib H46, but rib-protecting clip N1000 can attach to the cranial margin H54 of the caudal rib H47 in similar fashion.

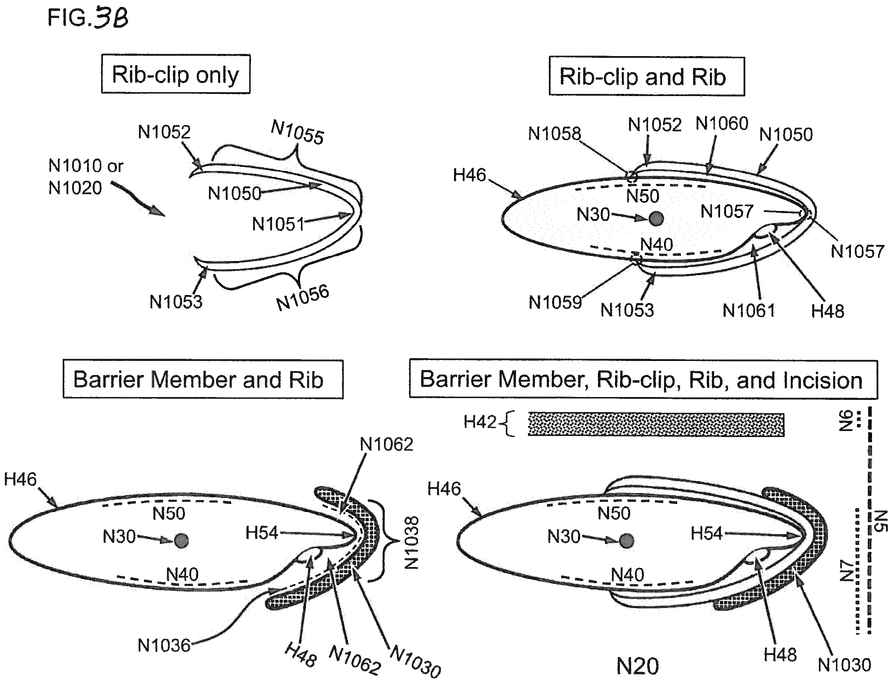

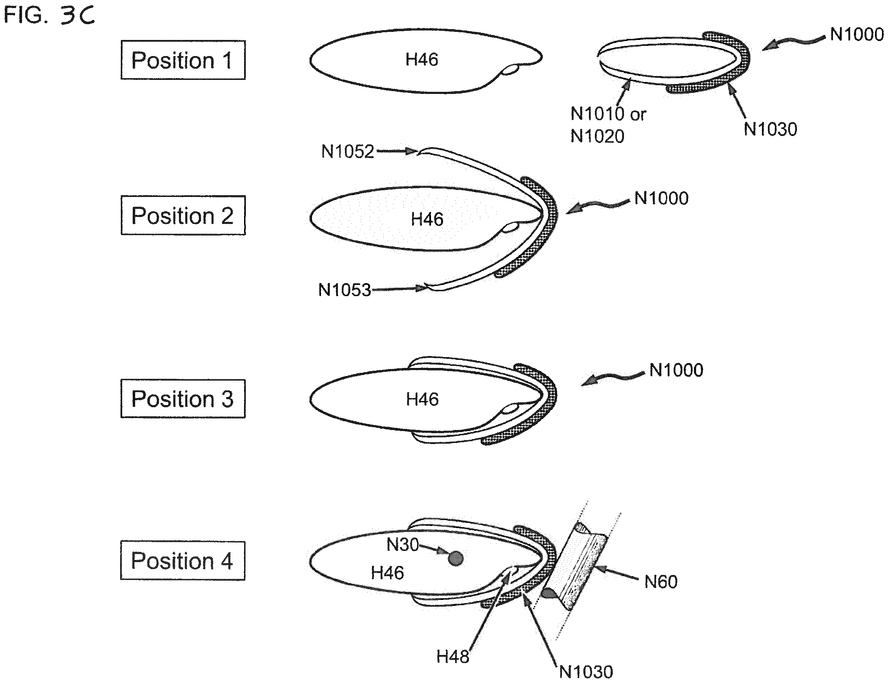

Rib-protecting clip N1000 is comprised of a rib-engaging channel N1005 formed by a first rib-clip N1010, a second rib-clip N1020, and a barrier member N1030. FIG 3A shows an oblique view of rib-protecting clip N1000 apposed to cranial rib H46 generally along the longitudinal axis N30 of cranial rib H46. FIG 3B shows four panels, each presenting a cross-section through rib-protecting clip N1000. Panel "Rib-clip Only" shows a rib-clip N1010 or N1020 in isolation in side view. Panel "Rib-clip and Rib" shows a rib-clip N1010 or N1020 and cranial rib H46 in cross section. Panel "Barrier Member and Rib" shows a cross-section through cranial rib H46 and a portion of barrier member N1030 such that barrier member N1030 is seen in isolation with cranial rib H46. Panel "Barrier Member, Rib-clip, Rib, and Incision" shows a cross section through rib-protecting clip N1000 through one end of barrier member N1030 such that all components are seen in cross-section. FIG. 3C shows a sequence of illustrations depicting how rib-protecting clip N1000 is applied to a rib.

Barrier member N1030 is preferably an elongated plate curved as shown here, but can be any elongated component such as a cylindrical bar. Barrier member N1030 has a first end N1032, a second end N1034, a rib-facing surface N1036, and an incision-facing surface N1038. The length N1031 of barrier member N1030 is thus the distance separating first end N1032 and second end N1034. First rib-clip N1010 is disposed on rib-facing surface N1036 at first end N1032, and second rib-clip N1020 is disposed on rib-facing surface N1036 at second end N1034. Barrier member N1030 can have one of several different lengths N1031 to accommodate different incision sizes. For example, barrier member N1030 can have a length N1031 ranging from 2 to 15 mm for the camera port for thoracoscopic surgery (fitting into a very small incision), or it can have a length N1031 of 100 mm for a utility incision for thoracoscopic surgery (permitting access for larger instruments or for multiple instruments simultaneously).

First rib-clip N1010 and second rib-clip N1020 have similar components. Each rib-clip N1010 or N1020 is comprised of bent elongated member N1050 that wraps cranial rib H46. Bent elongated member N1050 has an apex N1051, an upper end N1052, and a lower end N1053. Upper segment N1055 is that portion of bent elongated member N1050 that spans from apex N1051 to upper end N1052, and lower segment N1056 is that portion of bent elongated member N1050 that spans from apex N1051 to lower end N1053. Either or both ends N1052 and N1053 can be sharp or slightly blunted. Apex N1051 contacts the caudal margin H54 of cranial rib H46 at apex contact area N1057. Upper end N1052 contacts the skin surface N50 of cranial rib H46 at upper contact area N1058, and lower end N1053 contacts the pleural surface N40 of cranial rib H46 at lower contact area N1059. Apex contact area N1057, upper contact area N1058, and lower contact area N1059 are substantially away from the neurovascular bundle H48 of cranial rib H46. Upper segment N1055 and lower segment N1056 are curved such they create upper gap N1060 and lower gap N1061, respectively, and do not touch either cranial rib H46 or neurovascular bundle H48.

First and second rib clips N1010 and N1020 attach reversibly to cranial rib H46 and appose barrier member N1030 along the caudal margin H50 of cranial rib H46, but without barrier member N1030 touching cranial rib H46. Effectively, first rib clip N1010 and second rib clip N1020 act as a raised member (as described for FIGS. 2D and 2E). There is thus a barrier member gap N1062 between cranial rib H46 and the rib-facing surface N1036 of barrier member N1030. Barrier member N1030 thus does not crush tissue attached to cranial rib H46, but barrier member N1030 still functions as a shield to protect cranial rib H46 and any tissue lying in barrier member gap N1050. Upper gap N1060, lower gap N1061, and barrier gap N1062 thus prevent rib-protecting clip N1000 from contacting cranial rib anywhere but at the apex contact area N1057, upper contact area N1058, and lower contact area N1059 for first rib clip N1010 and for second rib clip N1020.

FIG. 3C shows a sequence of illustrations depicting how rib-protecting clip N1000 is applied to a rib. Rib-protecting clip N1000 can be made of metal or polymer or any material with sufficient rigidity and toughness to (a) elastically deform and (b) hold instruments away from cranial rib H46. In Position 1, rib-protecting clip N1000 is separate from cranial rib H46. In Position 2, rib-protecting clip N1000 has been deformed to separate upper end N1052 from lower end N1053, and rib-protecting clip N1000 is inserted onto cranial rib H46 until it contacts the caudal margin H50 of the cranial rib H46. The actions of deforming rib-protecting clip N1000 and placing it onto cranial rib H46 can be performed by hand or with the assistance of a separate clip applier for this purpose. In Position 3, rib-protecting clip N1000 has been allowed to elastically return to its undeformed shape, and now upper end N1052 and lower end N1053 are in contact with cranial rib H46, holding barrier member N1030 firmly in place, aligned generally along the longitudinal axis N30 of the cranial rib H46. Barrier member N1030 is now firmly held in position against cranial rib H46, creating upper gaps 1060, lower gaps, 1061, and barrier member gap N1062 and shielding cranial rib H46 and neurovascular bundle H48 from impingement by surgical instrument N60.

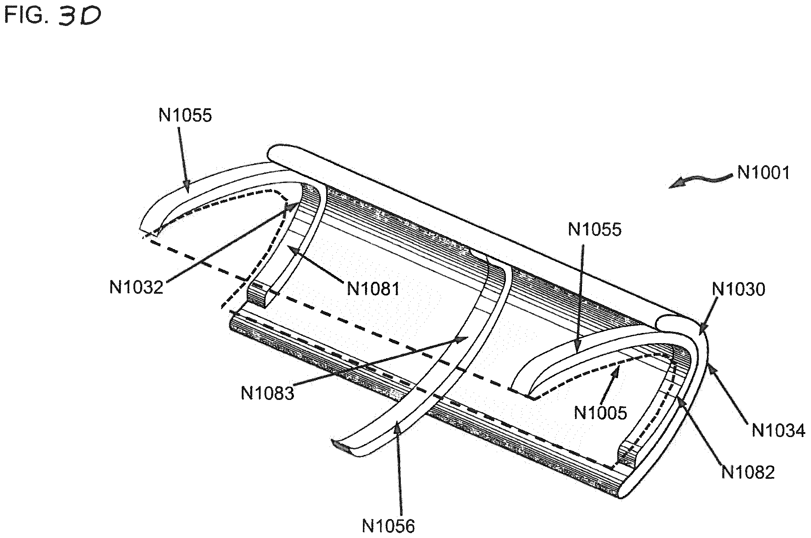

A rib-protecting clip, like rib-protecting clip N1000, can be configured in several ways. Barrier member N1030 can instead be supported by a multiple rib-clips with some disposed between first end N1032 and second end N1034 of barrier member N1030, which would help support a barrier member N1030 with longer length N1031. Similarly, the rib-clips need not have both an upper segment N1055/upper end N1052 and a lower segment N1056/lower end N1053. For example, the rib-protecting clip N1001 in FIG. 3D is comprised of a barrier member N1030, a first rib-clip N1081 on first end N1032 of barrier member N1030, a second rib-clip N1082 on second end N1034 of barrier member N1030, and a third rib-clip N1083 halfway between first end N1032 and second end N1034. First rib-clip N1081 and second rib-clip N1082 have only an upper segment N1055/upper end N1052 while third rib-clip N1083 has only a lower segment N1056/lower end N1053. These three ends of the three clips would comprise a "3-point rib clip" connected by the barrier member N1030, and rib-engaging channel N1005 is formed by first rib-clip N1110, second rib-clip N1120, third rib-clip N1130 and barrier member N1030.

The invention is not to be limited to the specific embodiments disclosed and modifications and other embodiments are intended to be included within the scope of the appended claims. Although specific terms are used herein, they are used in a generic and descriptive sense only and not for the purposes of limitation.

## Claims

1. A rib-protecting clip assembly (N900, N1000) for thoracic surgical access through an intercostal incision (N7) comprising:
a clip (N1030) having a clip body (N902, N1032) configured to fit substantially entirely within the intercostal incision (N7);
at least one rib-engaging channel (N901, N1005) formed in the clip body, the rib-engaging channel having a first end (N902, N1032) and a second end (N903, N1034); **characterized in that** the clip assembly further comprises:
at least one raised member (N910, N1010), wherein the raised member is:
disposed along a portion of the rib-engaging channel at or between the first and second ends of the rib-engaging channel;
elevated from the rib engaging channel and configured to extend toward a rib (H46); and
disposed along a portion of the rib engaging channel such that:
at least one rib-contacting surface on the at least one raised member (N910, N1010) is arranged to, in use, contact at least one rib in order to reduce or remove the pressure that may otherwise be placed on the rib and associated intercostal muscle by the remainder of the rib engaging channel (N901, N1005).

2. The rib-protecting clip assembly of Claim 1 having at least two spaced apart raised members (N901: N1010, N1020), one at or towards each respective one of the first and second ends of the rib-engaging channel.

3. The rib-protecting clip assembly of claim 2 wherein the distance between the raised members (N901: N1010, N1020) is greater than the height of the clip assembly.

4. The rib-protecting clip assembly of claim 1 or claim 2 wherein the clip (N910) has a rib-facing channel (N901, N1005) and the raised member (N910) or members (N910) is/are on the rib-facing channel.

5. The rib-protecting clip assembly of claim 4 wherein the clip (N901, N1030) has a height greater than the thickness of a rib (H46) which it is intended to in use protect, with the rib-facing channel (N901, N1005) being arranged to wrap around a caudal margin of the rib (H46).

6. The rib-protecting clip assembly of any one of claims 1 to 4 comprising at least two raised members (N1010, N1020) each in the form of a rib clip (N1010, N1020) disposed on a rib facing surface of the clip (N1030) each comprised of a curved elongate member having an apex (N1051) and arranged to, in use, wrap over and contact the rib (H46) at two or more of an apex contact area (N1057) adjacent to the clip (N1030), an upper contact area (N1058), or a lower contact area (N1057), all located substantially away from the neurovascular bundle (H48) of the rib (H46).

7. The rib-protecting clip assembly of claim 6 comprising two rib clips (N1010, N1020) which each contact an upper contact area (N1058), an apex contact area (N1057) and a lower contact area (N1057) and thus each define three points of contact.

8. The rib-protecting clip assembly of claims 6 or 7 comprising at least three rib clips, a middle rib clip (N1056) which is arranged to contact an apex contact area and one only of an upper or lower contact area, the middle rib clip (N1056) being located between two further rib clips (N1055, N1055) which are each arranged to contact an apex contact area and one only of a lower contact area and an upper contact area different to the contact area contracted by the middle rib clip (N1056).

9. The rib-protecting clip assembly of claims 6, 7, 8 or 9 wherein the clip assembly is elastically deformable.

10. The rib-protecting clip assembly of claims 6, 7, 8, 9 or 10 wherein the rib clips are elastically deformable.

11. The rib-protecting clip assembly of any proceeding claim wherein the clip is elongate and arranged to be retained in place, in use, so that it is aligned substantial parallel with the rib to which it is clipped.

## Patentansprüche

1. Eine Rippenschutzclip-Baugruppe (N900, N1000) für Thoraxchirurgiezugang durch einen Interkostalschnitt (N7), die Folgendes beinhaltet:
einen Clip (N1030) mit einem Clip-Hauptteil (N902, N1032), der dazu ausgelegt ist, im Wesentlichen ganz in den Interkostalschnitt (N7) hinein zu passen;
mindestens einen Rippeneingriffskanal (N901, N1005), der in dem Clip-Hauptteil ausgebildet ist, wobei der Rippeneingriffskanal ein erstes Ende (N902, N1032) und ein zweites Ende (N903, N1034) aufweist;
**dadurch gekennzeichnet, dass** die Clip-Baugruppe ferner Folgendes beinhaltet:
mindestens ein erhabenes Element (N910, N1010), wobei das erhabene Element:
entlang eines Anteils des Rippeneingriffskanals an den ersten und zweiten Enden oder zwischen dem ersten und zweiten Ende des Rippeneingriffskanals angeordnet ist;
von dem Rippeneingriffskanal erhöht ist und dazu ausgelegt ist, sich zu einer Rippe (H46) hin zu erstrecken; und
entlang eines Anteils des Rippeneingriffskanals angeordnet ist, so dass:
mindestens eine Rippenkontaktfläche auf dem mindestens einen erhabenen Element (N910, N1010)so angeordnet ist, dass sie im Gebrauch mit mindestens einer Rippe in Kontakt kommt, um den Druck, der ansonsten auf der Rippe und dem dazugehörigen Interkostalmuskel durch den restlichen Rippeneingriffskanal (N901, N1005) lasten würde, zu reduzieren oder zu entfernen.

2. Die Rippenschutzclip-Baugruppe gemäß Anspruch 1, die mindestens zwei beabstandete erhabene Elemente (N901: N1010, N1020), eines an dem jeweiligen von dem ersten und zweiten Ende des Rippeneingriffskanals oder zu diesem hin, aufweist.

3. Die Rippenschutzclip-Baugruppe gemäß Anspruch 2, wobei der Abstand zwischen den erhabenen Elementen (N901: N1010, N1020) größer ist als die Höhe der Clip-Baugruppe.

4. Die Rippenschutzclip-Baugruppe gemäß Anspruch 1 oder Anspruch 2, wobei der Clip (N910) einen rippenseitigen Kanal (N901, N1005) aufweist und das erhabene Element (N910) liegt/die erhabenen Elemente (N910) liegen auf dem rippenseitigen Kanal.

5. Die Rippenschutzclip-Baugruppe gemäß Anspruch 4, wobei der Clip (N901, N1030) eine Höhe aufweist, die größer ist als die Dicke einer Rippe (H46), zu deren Schutz er im Gebrauch vorgesehen ist, wobei der rippenseitige Kanal (N901, N1005) so angeordnet ist, dass er um einen Kaudalrand der Rippe (H46) wickelt wird.

6. Die Rippenschutzclip-Baugruppe gemäß einem der Ansprüche 1 bis 4, die mindestens zwei erhabene Elemente (N1010, N1020) beinhaltet, von denen jedes die Form eines Rippenclips (N1010, N1020) hat, der auf einer rippenseitigen Oberfläche des Clips (N1030) angeordnet ist, wobei jeder aus einem gekrümmten, länglichen Element besteht, das einen Scheitelpunkt (N1051) aufweist und dazu angeordnet ist, im Gebrauch über die Rippe (H46) gewickelt zu werden und an zwei oder mehreren von einem Scheitelkontaktbereich (N1057) neben dem Clip (N1030), einem oberen Kontaktbereich (N1058) oder einem unteren Kontaktbereich (N1057), die sich alle im Wesentlichen weg von dem neurovaskulären Bündel (H48) der Rippe (H46) befinden, mit der Rippe in Kontakt zu stehen.

7. Die Rippenschutzclip-Baugruppe gemäß Anspruch 6, die zwei Rippenclips (N1010, N1020) beinhaltet, die jeweils mit einem oberen Kontaktbereich (N1058), einem Scheitelpunkt-Kontaktbereich (N1057) und einem unteren Kontaktbereich (N1057) in Kontakt stehen und somit jeweils drei Kontaktpunkte definieren.

8. Die Rippenschutzclip-Baugruppe gemäß Ansprüchen 6 oder 7, die mindestens drei Rippenclips beinhaltet, wobei ein mittlerer Rippenclip (N1056) dazu angeordnet ist, mit einem Scheitelpunkt-Kontaktbereich und nur einem von einem oberen oder unteren Kontaktbereich in Kontakt zu stehen, wobei der mittlere Rippenclip (N1056) sich zwischen zwei weiteren Rippenclips (N1055, N1055) befindet, die jeweils dazu angeordnet sind, mit einem Scheitelpunkt-Kontaktbereich und nur einem von einem unteren Kontaktbereich und einem oberen Kontaktbereich in Kontakt zu stehen, der sich von dem Kontaktbereich unterscheidet, mit dem der mittlere Rippenclip (N1056) in Kontakt steht.

9. Die Rippenschutzclip-Baugruppe gemäß Ansprüchen 6, 7, 8 oder 9, wobei die Clip-Baugruppe elastisch verformbar ist.

10. Die Rippenschutzclip-Baugruppe gemäß Ansprüchen 6, 7, 8, 9 oder 10, wobei die Rippenclips elastisch verformbar sind.

11. Die Rippenschutzclip-Baugruppe gemäß einem der vorhergehenden Ansprüche, wobei der Clip länglich ist und dazu angeordnet ist, im Gebrauch an Ort und Stelle festgehalten zu werden, so dass er im Wesentlichen parallel mit der Rippe ausgerichtet ist, an der er mit einem Clip befestigt ist.

## Revendications

1. Ensemble clip de protection des côtes (N900, N1000) permettant un accès chirurgical thoracique dans une incision intercostale (N7) comportant :
un clip (N1030) présentant un corps de clip (N902, N1032) configuré pour s'adapter sensiblement entièrement à l'intérieur de l'incision intercostale (N7) ;
au moins un canal de mise en prise des côtes (N901, N1005) formé dans le corps de clip, le canal de mise en prise des côtes présentant une première extrémité (N902, N1032) et une seconde extrémité (N903, N1034) ;
**caractérisé en ce que** l'ensemble clip comporte en outre :
au moins un élément surélevé (N910, N1010), dans lequel l'élément surélevé est :
disposé le long d'une partie du canal de mise en prise des côtes au niveau des première et seconde extrémités du canal de mise en prise des côtes ou entre celles-ci ;
surélevé par rapport au canal de mise en prise des côtes et configuré pour s'étendre en direction d'une côte (H46) ; et
disposé le long d'une partie du canal de mise en prise des côtes de telle sorte que :
au moins une surface de contact avec les côtes sur ledit au moins un élément surélevé (N910, N1010) est agencée pour, lors de l'utilisation, venir en contact avec au moins une côte afin de réduire ou d'éliminer la pression qui peut par ailleurs être exercée sur la côte et le muscle intercostal associé par le reste du canal de mise en prise des côtes (N901, N1005).

2. Ensemble clip de protection des côtes selon la revendication 1 présentant au moins deux éléments surélevés espacés l'un de l'autre (N901 : N1010, N1020), un au niveau ou en direction de chaque extrémité respective des première et seconde extrémités du canal de mise en prise des côtes.

3. Ensemble clip de protection des côtes selon la revendication 2 dans lequel la distance entre les deux éléments surélevés (N901 : N1010, N1020) est supérieure à la hauteur de l'ensemble clip.

4. Ensemble clip de protection des côtes selon la revendication 1 ou la revendication 2 dans lequel le clip (N910) présente un canal faisant face aux côtes (N901, N1005) et l'élément (N910) ou les éléments (N910) surélevés sont sur le canal faisant face aux côtes.

5. Ensemble clip de protection des côtes selon la revendication 4 dans lequel le clip (N901, N1030) a une hauteur supérieure à l'épaisseur d'une côte (H46) qu'il est destiné à protéger lors de l'utilisation, avec le canal faisant face aux côtes (N901, N1005) étant agencé pour envelopper une marge caudale de la côte (H46).

6. Ensemble clip de protection des côtes selon l'une quelconque des revendications 1 à 4 comportant au moins deux éléments surélevés (N1010, N1020), chacun sous forme de clip de côte (N1010, N1020) disposé sur une surface faisant face aux côtes du clip (N1030), chacun comportant un élément oblong courbe présentant un sommet (N1051) et agencé pour, lors de l'utilisation, recouvrir la côte (H46) et venir en contact avec celle-ci au niveau de deux zones de contact ou plus parmi une zone de contact de sommet (N1057) adjacente au clip (N1030), une zone de contact supérieure (N1058), ou une zone de contact inférieure (N1057), toutes étant situées sensiblement à distance du paquet vasculo-nerveux (H48) de la côte (H46).

7. Ensemble clip de protection des côtes selon la revendication 6 comportant deux clips de côte (N1010, N1020) qui viennent chacun en contact avec une zone de contact supérieure (N1058), une zone de contact de sommet (N1057) et une zone de contact inférieure (N1057) et délimitent ainsi chacun trois points de contact.

8. Ensemble clip de protection des côtes selon la revendication 6 ou 7 comportant au moins trois. clips de côte, un clip de côte moyen (N1056) qui est agencé pour venir en contact avec une zone de contact de sommet et l'une seulement d'une zone de contact supérieure ou une zone de contact inférieure, le clip de côte moyen (N1056) étant situé entre deux autres clips de côte (N1055, N1055) qui sont chacun agencés pour venir en contact avec une zone de contact de sommet et l'une seulement d'une zone de contact inférieure et d'une zone de contact supérieure différente de la zone de contact avec laquelle le clip de côte moyen (N1056) vient en contact.

9. Ensemble clip de protection des côtes selon les revendications 6, 7, 8 ou 9 dans lequel l'ensemble clip est élastiquement déformable.

10. Ensemble clip de protection des côtes selon les revendications 6, 7, 8, 9 ou 10 dans lequel les clips de côte sont élastiquement déformables.

11. Ensemble clip de protection des côtes selon l'une quelconque des revendications précédentes dans lequel le clip est oblong et agencé pour être maintenu en place, lors de l'utilisation, de façon à être aligné sensiblement parallèlement à la côte sur laquelle il est clipsé.
